# EUROPÄISCHE PATENTSCHRIFT

(11) **EP 0 679 378 B1**
(45) Veröffentlichungstag und Bekanntmachung des Hinweises auf die Patenterteilung: **24.01.1996**
(21) Anmeldenummer: 94103177.5
(22) Anmeldetag: 03.03.1994
(51) Int. Cl.: A61F 5/56

(54) **Vorrichtung zum Verhindern des Schnarchens**
Anti snoring device
Dispositif pour empêcher le ronflement

(43) Veröffentlichungstag der Anmeldung: 02.11.1995
(73) Patentinhaber: CCI CONSULTING AG, 8756 Mitlödi (CH)
(72) Erfinder: Dehnbostel, Sylvia, D-29223 Celle (DE)
(74) Vertreter: Patentanwälte Thömen & Körner

(56) Entgegenhaltungen:
- EP-A- 0 264 516
- WO-A-90/13276
- US-A- 3 132 647
- US-A- 4 669 459

## Beschreibung

Die Erfindung betrifft eine Vorrichtung zum Verhindern des Schnarchens nach dem Oberbegriff des Anspruchs 1.

Eine derartige Vorrichtung ist aus der DE 40 26 602 C1 bekannt. Die bekannte Vorrichtung umfaßt ein Prothesenteil, das an Zähnen des Benutzers befestigbar ist. Ein mittels einer Feder am Prothesenteil befestigter Bügel trägt an seinem in die Mundhöhle ragenden Ende ein Zungenteil, das einerseits die Zunge vom hinteren Gaumensegel wegdrücken kann und andererseits beim Schluckvorgang durch die Zunge an den Gaumen gedrückt werden kann. Dabei ist der Bügel im vorderen, zu den Schneidezähnen des Benutzers ragenden Bereich des Prothesenteils mittels einer Feder befestigt.

Die Wirkungsweise der bekannten Vorrichtung besteht darin, daß in der Schlafposition die Zunge mittels einer Pelotte mit individuell hergestellten Zungenteil vom hinteren Gaumensegel nach vorne gelenkt wird, so daß die Zunge nicht beim Ein- und Ausatmen an dem hinteren weichen Gaumensegel vibrieren und dadurch Geräusche erzeugen kann. Andererseits ermöglicht aber die federnde Anbringung des Bügels mit dem Zungenteil, daß dieses beim Schluckvorgang ausweichen kann, so daß beim Schlucken keine zu große Beeinträchtigung entsteht.

Es hat sich gezeigt, daß einzelne Benutzer besonders in der Gewöhnungsphase beim Tragen der Vorrichtung zu häufigem Schlucken mit starken Zungenbewegungen neigen, die ein zu weites nach vorn Schwenken des Bügels zur Folge hat. Aus dieser Lage kann der Bügel mit dem Zungenteil nicht wieder selbsttätig in diejenige Lage zurückkehren, in der er auf den hinteren Bereich der Zunge drückt und diese vom Gaumensegel fernhält. Die Vorrichtung ist dann wirkungslos.

Weiterhin ist die Ausführung einer den Bügel lagernden Feder aus Gründen der Sicherheit und Stabilität der Vorrichtung sowie der gewünschten Größe und Gleichmäßigkeit der Federbelastung ungünstig.

Der Erfindung liegt die Aufgabe zugrunde, eine Vorrichtung der im Oberbegriff des Anspruchs 1 beschriebenen Art dahingehend zu verbessern, daß die Sicherheit und Stabilität der Vorrichtung sowie die Größe und Gleichmäßigkeit der Kraftausübung auf den Bügel mit dem Zungenteil verbessert wird und daß ferner die Beibehaltung der für eine sichere Funktion der Vorrichtung erforderlichen Lage des Bügels mit dem Zungenteil gewährleistet ist.

Diese Aufgabe wird bei einer Vorrichtung gemäß dem Oberbegriff des Anspruchs 1 mit den im kennzeichnenden Teil angegebenen Merkmalen gelöst.

Durch die Verwendung einer Gaumenplatte wird erreicht, daß das Prothesenteil sicherer, fester und vor allem in einer definierten Lage in der Mundhöhle des Benutzers angeordnet und befestigt werden kann, so daß sich daraus auch eine definierte, stabile Lage für den Bügel mit dem Zungenteil ergibt. Weiterhin ist der Bügel mit abgewinkelten Gelenkansätzen schwenkbeweglich innerhalb der Gaumenplatte befestigt. Gegenüber der bekannten Ausführung ist er also nicht mehr mittels der Feder sondern unmittelbar am Prothesenteil befestigt und zwar in der Gaumenplatte. Dadurch ist gewährleistet, daß der Bügel sich auch bei großer Krafteinwirkung nicht lösen kann, so daß die Gefahr des Verschluckens oder der Aspiration loser Teile beseitigt ist.

Die Sicherheit gegen Lösen vom Teilen der Vorrichtung bezieht auch auf die Feder ein, welche ein flach in der Gaumenplatte eingebettetes Verankerungsteil und eine dazu abgewinkelte Schwinge besitzt. Durch die Lage des Verankerungsteils wird sichergestellt, daß die Feder sich ohne Ermüdungserscheinungen im Prothesenteil abstützen kann und so über die gesamte Benutzungsdauer der Vorrichtung eine gleichbleibende Belastung auf den Bügel mit dem Zungenteil ausüben kann.

Die in der Gaumenplatte angeordnete Verdrehsperre sorgt dafür, daß der Bügel mit dem Zungenteil nicht in Richtung der Schneidezähne hinausschwenken kann, aus welcher Lage er von selbst nicht wieder in diejenige Position zurückkehren würde, in der er auf den hinteren Teil der Zunge drücken kann. Vielmehr wird sichergestellt, daß auch bei starkem Zungenschlag der Bügel maximal eine senkrechte Lage zur Gaumenplatte einnehmen kann, so daß er nach Schluckvorgängen automatisch wieder in die Position zurückkehren und die Zunge vom Gaumensegel wegdrücken kann.

Bei einer praktischen Ausgestaltung besteht die Verdrehsperre aus einem in der Gaumenplatte eingebetteten Verankerungsteil und einem dazu abgewinkelten, aus der Gaumenplatte vorstehenden Anschlagteil für den Bügel.

Das Verankerungsteil sorgt dafür, daß die Verdrehsperre auch großen mit der Zunge ausgeübten Kräften standhalten kann, wenn der aus der Gaumenplatte vorstehende Anschlagteil durch den Druck des Bügels belastet wird.

In vorteilhafter Ausgestaltung der Vorrichtung ist der Bügel im mittleren Bereich des Daches der Gaumenplatte befestigt.

Gegenüber der bekannten Vorrichtung, bei der Bügel in einem Prothesenteil nahe den Schneidezähnen befestigt ist, führt die Befestigung im mittleren Bereich des Daches der Gaumenplatte zu einer steileren Winkelstellung des Bügels relativ zur Gaumenplatte. Eine bezogen auf die bekannte Vorrichtung gleiche Kraftkomponente, die die Zunge vom Gaumensegel abhebt und nach vorn lenkt, kann dabei mit einer wesentlich geringeren Andruckkraft des am Ende des Bügels angebrachten Zungenteils auf die Zunge erreicht werden. Der mit dieser Maßnahme erzielte Effekt erleichtert dem Benutzer das Schlucken, da die Zunge beim Schlucken gegen den Gaumen gehoben wird und dabei auch die Andruckkraft des Zungenteils überwinden muß.

Eine Weiterbildung sieht vor, daß der Bügel zwei Schenkel umfaßt, deren Abstand sich, ausgehend von den abgewinkelten Gelenkansätzen bis zur Aufspreizung in eine das Zungenteil lagernde Schleife, stetig vermindert.

Bei dieser Ausführung ergibt sich eine bessere Seitenstabilität, so daß bei von der Zunge ausgeübten Seitenkräften diese von den nunmehr im Winkel angestellten Schenkeln besser gekontert werden können.

Bei einer bevorzugten Ausgestaltung ist die Gaumenplatte über die Verbindungslinie zwischen den hinteren Kanten der letzten Backenzähne, der sogenannten AH-Linie, hinaus verlängert.

Es wurde herausgefunden, daß diese Maßnahme zusätzlich zu einer Verminderung der Schnarchgeräusche beiträgt. Dies beruht unter anderem darauf, daß die Schleimhäute im Rachenbereich abgestützt werden, was einer Tendenz zum Vibrieren entgegenwirkt. Weiterhin wirkt sich die Berührung der im Rachenbereich befindlichen Schleimhäute durch die Verlängerung der Gaumenplatte als Refleximpuls aus, der ein Erschlaffen der Schleimhäute verhindert. Durch aktive Straffung der Schleimhäute wird der vibrationshemmende Effekt zusätzlich verstärkt.

Gemäß einer Weiterbildung weist das Prothesenteil seitliche Ansätze auf, die sich an die Gaumenplatte anschließen, an den inneren Flanken der Backenzähne ansteigen, zu den Kauflächen abgewinkelt sind und die Kauflächen als künstliche Überhöhung überlappen.

Die künstliche Überhöhung der Kauflächen bewirkt eine veränderte Position des Unterkiefers, die sich ebenfalls günstig auf eine Reduzierung der Schnarchgeräusche auswirkt. Zum Verständnis dieser Maßnahme ist zunächst zu beachten, daß ein Schläfer in der entspannten Situation des Schlafens eine Ruheschwebelage von ca. 2 bis 3 mm schwebendem Abstand zwischen den Zahnreihen einnimmt und dabei der Unterkiefer eine etwas nach hinten fallende Position einnimmt. Diese Position begünstigt es, die Zunge im erschlafften Zustand nach hinten fallen zu lassen, um hier am Gaumensegel zu flattern.

Die künstliche Erhöhung der Kauflächen bewirkt einen Reiz auf den Unterkiefer, so daß dieser wieder nach vorne kommt, um die Schlußbißstellung zu erreichen. Dies führt über den Bügel und den Druck des Zungenteils auf die Zunge zu einer Rückstellung der Zunge in eine vorgelagerte Position. Im übrigen ermöglichen die die Kauflächen überlappenden Ansätze eine okklusale Adjustierung, die außerdem einer statischen Stabilisierung des Prothesenteils in der Mundhöhle dient und dabei eine unerwünschte Regulierung des Gebisses verhindern hilft.

Gemäß einer Weiterbildung weist das Prothesenteil Halteelemente auf, die durch die an den inneren Flanken der Backenzähne ansteigenden und anliegenden Ansätze sowie durch an den gegenüberliegenden äußeren Flanken der Backenzähne anliegende Klammern gebildet sind.

Durch diese Ausgestaltung der Halteelemente werden Haltekräfte nicht durch einseitigen Druck auf die Zähne des Gebisses ausgeübt, sondern die Vorrichtung wird beidseitig an den inneren und äußeren Flanken der Backenzähne abgestützt, so daß sich die Befestigung der Vorrichtung neutral auf das Gebiß auswirkt und keine unerwünschten aktiven Regulierungseffekte auslöst.

Vorzugsweise sind die Klammern als Dreiecksklammern ausgebildet, die jeweils an zwei benachbarten Backenzähnen anliegen. Mit Hilfe dieser Dreiecksklammern läßt sich eine exakte Lage in der Mundhöhle sicherstellen, wobei gleichzeitig die erforderlichen Haltekräfte zur Festlegung am Gebiß aufgebracht werden können.

Ferner kann das Prothesenteil bei teilbezahnten Benutzern Zahnersatznachbildungen tragen.

Dadurch wird ein besserer Halt des Prothesenteils auf dem Restzahngebiß erzielt, eine unerwünschte Regulierung des Restzahngebisses vermieden und schließlich auch der optische Eindruck beim Tragen der Vorrichtung verbessert. Da die Vorrichtung ja nicht zum Kauen verwendet wird, reichen Zahnersatznachbildungen statt künstlicher Zähne, so daß für deren Herstellung das gleiche Material in Betracht kommt, das auch für das Prothesenteil verwendet wird.

Weiterbildungen und vorteilhafte Ausgestaltungen ergeben sich aus den Ansprüchen, der weiteren Beschreibung und der Zeichnung, die die Erfindung veranschaulichen.

In der Zeichnung zeigen:
- Fig. 1: eine im Gaumen eingesetzte Vorrichtung nach der Erfindung in Draufsicht;
- Fig. 2: einen Querschnitt durch die Vorrichtung entlang der Schnittlinie AA
- Fig. 3: einen Längsschnitt durch die Vorrichtung entlang der Schnittlinie BB und
- Fig. 4: ein vergrößert dargestelltes Detail aus Fig. 3.

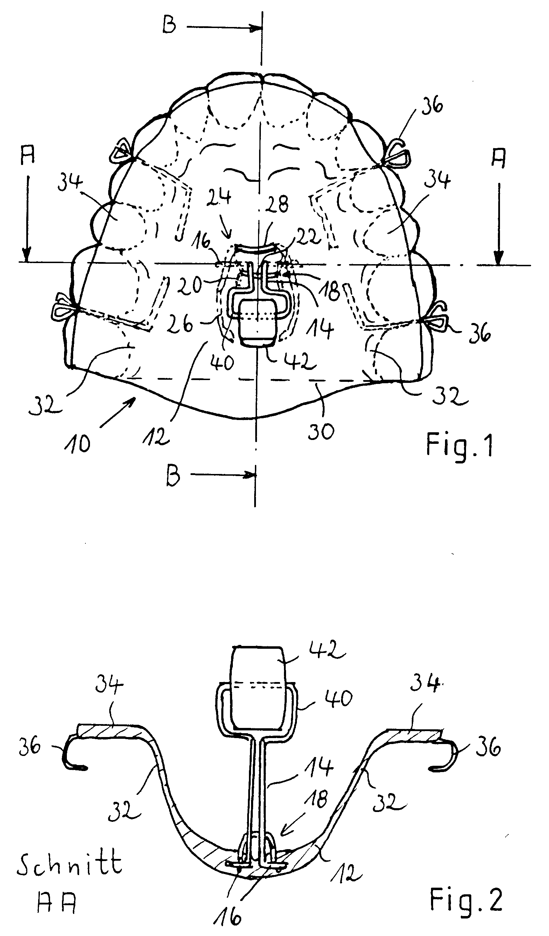

Fig. 1 zeigt eine Draufsicht auf die erfindungsgemäße im Oberkiefer eines Benutzers eingesetzte Vorrichtung. Die Vorrichtung umfaßt ein Prothesenteil 10, das an den Zähnen des Benutzers ähnlich einer Zahnprothese befestigbar ist. Das Prothesenteil 10 besitzt als Bestandteil eine Gaumenplatte 12, die am Gaumen des Benutzers anliegt, und innerhalb der ein Bügel 14 mit abgewinkelten Gelenkansätzen 16 schwenkbeweglich befestigt ist. Der bevorzugte Befestigungsort ist der mittlere Bereich des Daches der Gaumenplatte. Am Ende des Bügels 14, der dort als Rahmenteil 40 ausgebildet ist, befindet sich ein Zungenteil 42, das auf einer Achse des Rahmenteils 40 beweglich angebracht ist.

Eine Feder 18 aus einem flach in der Gaumenplatte 12 eingebetteten Verankerungsteil 20 und einer dazu abgewinkelten Schwinge 22 drückt unter Federkraft gegen den Bügel 14. Außerdem ist in der Gaumenplatte 12 eine Verdrehsperre 24 angeordnet, die die Schwenkbeweglichkeit des Bügels 14 in Richtung der Schneidezähne begrenzt. Die begrenzende Wirkung setzt bei einer senkrechten Lage des Bügels 14 gegenüber der Gaumenplatte 12 ein.

Die Verdrehsperre 24 besteht aus einem in der Gaumenplatte 12 eingebetteten Verankerungsteil 26 und einem dazu abgewinkelten, aus der Gaumenplatte 12 vorstehenden Anschlagteil 28 für den Bügel 14. Bezogen auf die Lage der Schneidezähne befindet sich das Anschlagteil 28 der Verdrehsperre 24 somit vor dem Bügel 14 und die Schwinge 22 der Feder 18 hinter dem Bügel 14. Die Federkraft ist so gerichtet, daß der Bügel 14 eine etwa senkrechte Lage zur Gaumenplatte 12 einzunehmen bestrebt ist.

Durch die Federkraft, mit der die Schwinge 22 gegen den Bügel 14 drückt, wird das am Ende des Bügels 14 befestigte Zungenteil 42 so auf die Zunge gedrückt, daß die Zunge vom hinteren Gaumensegel abgehoben ist. Dadurch wird im entspannten Zustand während des Schlafes eine Vibration der Zunge am Gaumensegel durch den beim Ein- und Ausatmen vorbeistreichenden Luftstrom vermieden. Wenn die Zunge beim Schlucken am Gaumen abrollt, kann der Bügel 14 mit dem Zungenteil 42 gegen die Kraft der Feder 18 ausweichen und dadurch den Schluckvorgang ohne wesentliche Behinderung ermöglichen. Durch die Drehbarkeit des Zungenteils 42 gegenüber dem Bügel 14 wird erreicht, daß auch bei sich ändernden Winkelstellungen des Bügels 14 das Zungenteil 42 flach auf der Zunge liegen kann.

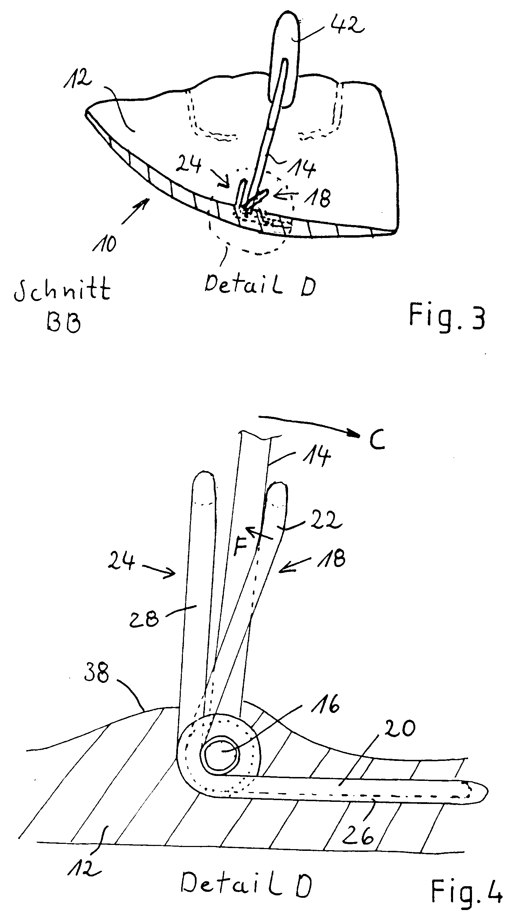

In Fig. 1 sind die aus der Gaumenplatte 12 vorstehenden Teile des Bügels 14, der Feder 18 und der Verdrehsperre 24 ausgezogen gezeichnet, während die innerhalb der Gaumenplatte befindlichen Teile gestrichelt gezeichnet sind. Zur Verdeutlichung der gegenseitigen Lage wird ergänzend auf die Querschnittszeichnung Fig. 2, die Längsschnittzeichnung Fig. 3 und speziell auf die das Detail D aus Fig. 3 vergrößert darstellende Zeichnung in Fig. 4 verwiesen.

Wie in Fig. 4 erkennbar, ist an der Stelle, an der die Gelenkansätze 16 des Bügels 14 innerhalb der Gaumenplatte 12 befestigt sind, ein Wulst 38 vorhanden, der jedoch in der Mitte zur Gewährleistung der Schwenkbeweglichkeit des Bügels 14 unterbrochen ist. Innerhalb des Wulstes 38 sind die Übergänge zwischen dem Verankerungsteil 20 und der Schwinge 22 der Feder 18 sowie die Übergänge zwischen dem Verankerungsteil 26 und dem Anschlagteil 28 der Verdrehsperre 24 unter Bildung eines Auges ein- oder mehrmals gewunden. Diese Windungen haben einen solchen Innendurchmesser, daß sie als Lager zur Aufnahme der Gelenkansätze 16 des Bügels 18 dienen. Dies hat bei der Herstellung der Vorrichtung den Vorteil, daß der Bügel 14 mit der Feder 18 und der Verdrehsperre 24 vormontiert und vorpositioniert werden kann und dann beim Gießen der Gaumenplatte 12 eingegossen und so in die Gaumenplatte 12 integriert werden kann. Die Einhaltung der die Funktion von Feder 18, Bügel 14 und Verdrehsperre 24 sichernden relativen Position zueinander sowie zum Prothesenteil 10 wird somit gewährleistet.

In Fig. 4 liegen der Verankerungsteil 26 der Verdrehsperre 24 und der Verankerungsteil 20 der Feder 18 hintereinander in derselben Ebene eingebettet in der Gaumenplatte 12. Da das Verankerungsteil 20 der Feder 18 einen kleineren Materialdurchmesser als das Verankerungsteil 26 der Verdrehsperre 24 besitzt, ist dies durch gestrichelte Darstellung kenntlich gemacht.

In Fig. 4 ist noch die beim Schluckvorgang erforderliche Schwenkbeweglichkeit des Bügels 14 durch den Pfeil C angedeutet und die Kraftrichtung, mit der die Feder 18 gegen den Bügel 14 drückt, durch den Pfeil F.

Aus der Schnittdarstellung in Fig. 2 wird deutlich, daß die beiden Schenkel des Bügels 14 ausgehend von den Gelenkansätzen 16 bis zu der Stelle, an der sie sich zur Schleife 40 spreizen, im Abstand stetig verringert sind. Es ergibt sich dadurch eine verbesserte Seitenstabilität.

Die Integration der Gelenkansätze 16 zusammen mit den Verankerungsteilen 20 und 26 von Feder und Verdrehsperre 24 in der Gaumenplatte 12 sorgt dafür, daß eine präzise Lagerung des Bügels 14 ermöglicht wird und daß die Feder lediglich noch die Andruckkraft auf den Bügel 14 aufbringen, im Gegensatz zum Stand der Technik diesen jedoch nicht mehr lagern muß. Die Gefahr, daß sich beim Bruch der Feder der gesamte Bügel mit dem Zungenteil löst und verschluckt werden kann, ist dadurch beseitigt. Die Verdrehsperre 24 sorgt dafür, daß der Bügel 14 auch bei starkem Zungenschlag, wie er bei Benutzern in der Gewöhnungsphase an die Vorrichtung besonders häufig auftritt, nicht in Richtung der Schneidezähne verschwenkt werden kann. In der vorderen Position könnte er nämlich nicht mehr selbsttätig in die hintere Lage zurückkehren und durch Druck auf die Zunge diese vom Gaumensegel abhalten. Die Funktion der Vorrichtung wäre in diesem Falle gestört und die Frontzähne durch dieses Hindernis bei entsprechendem Kaudruck frakturgefährdet.

Wie aus Fig. 1 weiter hervorgeht, bedeckt die Gaumenplatte 12 den gesamten Gaumen inklusive des weichen Gaumensegels und ist über die als AH-Linie bezeichnete Verbindungslinie 30 zwischen den hinteren Backenzähnen hinaus verlängert. Durch Reizung und Einschränkung der Vibrationsmöglichkeit der hinteren Schleimhäute trägt diese Maßnahme wesentlich zu einer weiteren Senkung der beim Schlafen noch vorhandenen Atemgeräusche und des Restschnarchens bei.

Wie Fig. 1 in Verbindung mit Fig. 2 erkennen läßt, weist das Prothesenteil 10 seitliche Ansätze 32 auf, die sich an die Gaumenplatte 12 anschließen. Die Ansätze 32 steigen an den inneren Flanken der Backenzähne an und sind zu den Kauflächen abgewinkelt. Sie überlappen die Kauflächen der Backenzähne und sind dort als künstliche Überhöhung 34 ausgebildet.

Durch diese künstliche Überhöhung 34 nimmt der Unterkiefer statt der im entspannten Zustand des Schlafens auftretenden nach hinten fallenden Position, die ebenfalls das Schnarchgeräusch begünstigt, wieder eine nach vorn kommende Position ein, um die Position der Schlußbißstellung zu erreichen. Dies wird zusätzlich auf die Zunge durch den Bügel 14 mit Druck über das Zungenteil 42 auf die Zunge übertragen und führt so auch zu der Rückstellung der Zunge in eine vorgelagerte Position.

Die als okklusale Adjustierung wirkenden Ansätze 32 mit den Überhöhungen 34 dienen außerdem der statischen Stabilisierung der Vorrichtung in der Mundhöhle, da durch den nun vorhandenen Kontakt der beiden Zahnreihen zueinander ein Verrutschen der Vorrichtung in eine andere Position ausgeschlossen ist.

Für einen weiteren Halt des Prothesenteils 10 sorgen Halteelemente, die einerseits durch die an den inneren Flanken der Backenzähne ansteigenden und anliegenden Ansätze 32 sowie durch Klammern 36 gebildet sind, die an den gegenüberliegenden äußeren Flanken der Backenzähne anliegen. Bei diesen Klammern handelt es sich vorzugsweise um Dreiecksklammern, die an den Außenflanken jeweils zweier benachbarter Backenzähne anliegen und neben einer Federwirkung auch die Einhaltung einer genauen Lage ermöglichen. Durch die Wechselwirkung der Klammern 36 und der von innen gegen die Flanken der Backenzähne drückenden Ansätze 32 werden die Backenzähne keinen Kräften ausgesetzt, die zu einer unerwünschten Zahnregulierung führen würden. Vielmehr sind die Kräfte gegeneinander aufgehoben und die Vorrichtung wird kraftneutral an den Zähnen befestigt.

Bei teilbezahnten Benutzern könnte das Prothesenteil auch an den Stellen fehlender eigener Zähne Zahnersatznachbildungen tragen. Hiermit wäre wiederum eine genaue Lage in der Mundhöhle gewährleistet und zum anderen wäre die Gefahr unerwünschter Veränderungen des Restzahngebisses gemindert. Abgesehen davon ergibt sich auch eine bessere optische Erscheinung beim Tragen der Vorrichtung, so daß nicht mehr ohne weiteres bemerkt werden kann, wenn der Benutzer eine Zahnprothese und alternativ dazu die erfindungsgemäße Vorrichtung trägt. Diese Vorgehensweise ist auf alle Patientenfälle von Vollbezahnung bis Vollprothese anwendbar.

## Patentansprüche

1. Vorrichtung zum Verhindern des Schnarchens, mit einem Prothesenteil, das an Zähnen des Benutzers befestigbar ist, einem Bügel und einem am in die Mundhöhle ragenden Ende des Bügels beweglich angebrachten Zungenteil, wobei der Bügel mittels einer Feder derart belastet ist, daß einerseits das Zungenteil die Zunge vom hinteren Gaumensegel wegdrücken kann und andererseits die Zunge beim Schluckvorgang den Bügel mit dem Zungenteil an den Gaumen drücken kann, dadurch gekennzeichnet, daß das Prothesenteil (10) eine am Gaumen des Benutzers anliegende Gaumenplatte (12) umfaßt, daß der Bügel (14) mit abgewinkelten Gelenkansätzen (16) schwenkbeweglich innerhalb der Gaumenplatte (12) befestigt ist, daß die Feder (18) aus einem flach in der Gaumenplatte (12) eingebetteten Verankerungsteil (20) und einer dazu abgewinkelten, unter Federkraft gegen den Bügel (14) drückenden Schwinge (22) besteht und daß in der Gaumenplatte (12) eine Verdrehsperre (24) angeordnet ist, die die Schwenkbeweglichkeit des Bügels (14) in Richtung der Schneidezähne über eine zur Gaumenplatte (12) senkrechte Lage hinaus begrenzt.

2. Vorrichtung nach Anspruch 1, dadurch gekennzeichnet, daß die Verdrehsperre (24) aus einem in der Gaumenplatte (12) eingebetteten Verankerungsteil (26) und einem dazu abgewinkelten, aus der Gaumenplatte (12) vorstehenden Anschlagteil (28) für den Bügel (14) besteht.

3. Vorrichtung nach Anspruch 1 oder 2, dadurch gekennzeichnet, daß der Bügel (14) im mittleren Bereich des Daches der Gaumenplatte (12) befestigt ist.

4. Vorrichtung nach einem der Ansprüche 1 bis 3, dadurch gekennzeichnet, daß der Bügel (14) zwei Schenkel umfaßt, deren Abstand sich, ausgehend von den abgewinkelten Gelenkansätzen (16) bis zur Aufspreizung in eine das Zungenteil (42) lagernde Schleife (40), stetig vermindert.

5. Vorrichtung nach einem der Ansprüche 1 bis 4, dadurch gekennzeichnet, daß die Gaumenplatte (12) über die Verbindungslinie (30) zwischen den hinteren Kanten der letzten Backenzähne, die AH-Linie, hinaus verlängert ist.

6. Vorrichtung nach einem der Ansprüche 1 bis 5, dadurch gekennzeichnet, daß das Prothesenteil (10) seitliche Ansätze (32) aufweist, die sich an die Gaumenplatte (12) anschließen, an den inneren Flanken der Backenzähne ansteigen, zu den Kauflächen abgewinkelt sind, und die Kauflächen als künstliche Überhöhung (34) überlappen.

7. Vorrichtung nach einem der Ansprüche 1 bis 6, dadurch gekennzeichnet, daß das Prothesenteil (10) Halteelemente umfaßt, die durch die an den inneren Flanken der Backenzähne ansteigenden und anliegenden Ansätze (32) sowie durch an den gegenüberliegenden äußeren Flanken der Backenzähne anliegende Klammern (36) gebildet sind.

8. Vorrichtung nach Anspruch 7, dadurch gekennzeichnet, daß die Klammern (36) als Dreiecksklammern ausgebildet sind, die jeweils an zwei benachbarten Backenzähne anliegen.

9. Vorrichtung nach einem der Ansprüche 1 bis 8, dadurch gekennzeichnet, daß das Prothesenteil bei teilbezahnten Benutzern Zahnersatznachbildungen trägt.

## Claims

1. Device for preventing snoring, comprising a prosthesis component which can be fixed to the teeth of the user, a stirrup and a tongue component movably mounted on the end of the stirrup penetrating into the oral cavity, wherein the stirrup can be loaded by means of a spring in such a manner that, on the one hand, the tongue component can press the tongue away from the rear soft palate and, on the other hand, during the swallowing action the tongue can press the stirrup with the tongue component onto the palate, characterized in that the prosthesis component (10) comprises a dental plate (12) bearing against the palate of the user, that the stirrup (14) is secured pivotally movable inside the dental plate (12) by cranked hinge attachments (16), that the spring (18) consists of an anchorage component (20) embedded flat in the dental plate (12) and of a rocker arm (22), cranked away therefrom and pressing under spring force against the stirrup (14), and that in the dental plate (12) a rotation barrier (24) is mounted, which limits the pivotal mobility of the stirrup (14) towards the incisors beyond a position perpendicular to the dental plate (12).

2. Device according to Claim 1, characterized in that the rotation barrier (24) consists of an anchorage component (26) embedded in the dental plate (12) and of an abutment component (28) for the stirrup (14), cranked away from the anchorage component and projecting out of the dental plate (12).

3. Device according to Claim 1 or 2, characterized in that the stirrup (14) is fixed in the central region of the roof of the dental plate (12).

4. Device according to one of Claims 1 to 3, characterized in that the stirrup (14) comprises two arms, the spacing between which, starting from the cranked hinge attachments (16), continually decreases until they spread out into a loop (40) which seats the tongue component (42).

5. Device according to one of Claims 1 to 4, characterized in that the dental plate (12) is continued beyond the connecting line (30) between the rear edges of the last molars, the AH-line.

6. Device according to one of Claims 1 to 5, characterized in that the prosthesis component (10) possesses lateral attachments (32), which adjoin the dental plate (12), rise along the inner flanks of the molars, are cranked onto the chewing surfaces, and overlap the chewing surfaces as an artificial crown (34).

7. Device according to one of Claims 1 to 6, characterized in that the prosthesis component (10) comprises holding elements, which are formed by the attachments (32) rising along and bearing against the inner flanks of the molars and of clips (36), bearing against the opposite, outer flanks of the molars.

8. Device according to Claim 7, characterized in that the clips (36) are constructed as triangular clips, which each bear against two adjacent molars.

9. Device according to one of Claims 1 to 8, characterized in that the prosthesis component carries imitation dentures in the case of users with missing teeth.

## Revendications

1. Dispositif permettant d'empêcher le ronflement, comprenant un élément de prothèse qui peut être fixé sur les dents de l'utilisateur, un étrier et une pièce linguale montée mobile sur l'extrémité de l'étrier en saillie dans la cavité buccale, l'étrier étant sollicité par un ressort de manière que, d'une part, la pièce linguale puisse éloigner la langue du voile arrière du palais et que, d'autre part, la langue puisse, pendant la déglutition, presser l'étrier, avec la pièce linguale, contre le palais, caractérisé en ce que l'élément de prothèse (10) comprend une plaque palatale (12) qui repose contre le palais de l'utilisateur, que l'étrier (14) est fixé dans la plaque palatale (12) de manière pivotante au moyen de parties coudées articulées (16), que le ressort (18) est constitué d'une pièce d'ancrage (20) plate insérée dans la plaque palatale (12) et d'une aile (22) formant un coude avec elle et, par effet de ressort, exerçant une pression sur l'étrier (14) et en ce que, dans la plaque palatale (12), est disposé un blocage anti-torsion (24) qui limite le mouvement pivotant de l'étrier (14) en direction des incisives en passant par une position perpendiculaire à la plaque palatale (12).

2. Dispositif selon la revendication 1, caractérisé en ce que le blocage anti-torsion (24) est constitué d'une pièce d'ancrage (26) insérée dans la plaque palatale (12) et d'une pièce de butée (28) pour l'étrier (14) en saillie sur la plaque palatale (12) et formant un coude avec la pièce d'ancrage (26).

3. Dispositif selon la revendication 1 ou 2, caractérisé en ce que l'étrier (14) est fixé dans la zone du milieu du sommet de la plaque palatale (12).

4. Dispositif selon une des revendications 1 à 3, caractérisé en ce que l'étrier (14) comprend deux branches dont la distance de l'une à l'autre va continuellement en diminuant à partir des parties coudées articulées (16) jusqu'à l'endroit où elles sont écartées pour former une boucle (40) qui reçoit la pièce linguale (42).

5. Dispositif selon une des revendications 1 à 4, caractérisé en ce que la plaque palatale (12) est prolongée au-delà de la ligne de jonction (30), entre les arêtes arrière des dernières molaires, la ligne AH.

6. Dispositif selon une des revendications 1 à 5, caractérisé en ce que l'élément de prothèse (10) présente des prolongements latéraux (32) qui se raccordent à la plaque palatale (12), qui montent sur les flancs intérieurs des molaires, qui sont coudées vers les surfaces de mastiquage et se superposent à celles-ci par un sur-haussement artificiel (34).

7. Dispositif selon une des revendications 1 à 6, caractérisé en ce que l'élément de prothèse (10) présente des supports formés par les prolongements (32) qui montent sur les flancs intérieurs des molaires et qui reposent contre elles, ainsi que par des crochets (36) qui reposent contre les flancs opposés extérieurs des molaires.

8. Dispositif selon la revendication 7, caractérisé en ce que les crochets (36) sont des crochets triangulaires qui reposent respectivement contre deux molaires voisines.

9. Dispositif selon une des revendications 1 à 8, caractérisé en ce que l'élément de prothèse peut comporter une denture partielle reproduite remplaçant les dents qui manquent à certains utilisateurs.
